# EUROPEAN PATENT SPECIFICATION

(11) **EP 3 179 963 B1**
(45) Date of publication and mention of the grant of the patent: **14.04.2021**
(21) Application number: 15832394.9
(22) Date of filing: 14.08.2015
(51) Int. Cl.: A61B 17/16, A61B 10/02, A61B 17/00

(54) **PROSTHETIC DEVICE**
PROTHESENVORRICHTUNG
DISPOSITIF PROTHÉTIQUE

(30) Priority: 15.08.2014 US 201414461097
(43) Date of publication of application: 21.06.2017
(73) Proprietor: Rocky Mountain Manufacturing LLC, Grand Junction, Colorado 81505 (US)
(72) Inventor: WILL, Michael W., Montrose, Colorado 81401 (US); LEWIS, Mark A., Grand Junction, Colorado 81505 (US)
(74) Representative: CSY London
(86) International application number: PCT/US2015/045334
(87) International publication number: WO 2016/025868

(56) References cited:
- US-A- 1 319 884
- US-A- 5 326 352
- US-A1- 2004 068 325
- US-A1- 2009 292 368
- US-A1- 2011 015 761
- US-B1- 6 312 475
- US-B2- 6 972 042
- US-B2- 8 641 780

## Description

### BACKGROUND

The present application generally relates to a prosthetic device. Prosthetic devices provide additional mobility to those persons with limited mobility due to a missing limb. These devices allow a recipient to accomplish those physical tasks of others not missing the limb.

The document US5326352 discloses apparatus for quickly attaching a leg prosthesis. US1319884 discloses a prosthetic hand which is adapted to be inserted into a socket attached to a wearer's arm.
US2009/0292368A1 discloses a prosthetic limb connector having a key to maintain an alignment with a connector body, the device of claim 1 differing from the device of this document in that the device further comprises a locking head which is coupled to a proximal end of the first conical part and is adapted to fit into a conical void defined in the upper member;
the conical terminating section comprises a first conical part which has a steeper angle than a second conical part relative to a common axis of the upper and lower members;
the guiding track is sloped and the guiding feature has a notch; the protrusion of the self-aligning feature extends perpendicular to the common axis of the upper and lower members;
contact of the self-aligning feature with the sloped guiding track as the conical terminating section is fitted into the conical void causes the lower member to rotate until the self-aligning feature is seated within the notch.

### BRIEF DESCRIPTION OF THE DRAWINGS

The accompanying drawings illustrate various examples of the principles described herein and are a part of the specification. The examples do not limit the scope of the claims.
Fig. 1 is a front elevation view of a prosthetic device according to one example of principles described herein.
Fig. 2 is a side elevation view of the upper member of the prosthetic device according to one example of principles described herein.
Fig. 3 is a top cross sectional view illustrating a prosthetic device locking feature according to one example of principles described herein.
Fig. 4 is a side elevation cross sectional view illustrating the internal workings of the prosthetic device according to one example of principles described herein.
Fig. 5 is a top view of the upper member (101) shown in Fig. 1 according to one example of the principles described herein.

Throughout the drawings, identical reference numbers designate similar, but not necessarily identical, elements.

### DETAILED DESCRIPTION

As briefly described above, prosthetic devices allow additional mobility for a recipient that would not otherwise be available to the amputee. Some prosthetic devices are awkward, expensive and not user friendly. Specifically, prosthetic foot and leg attachments are rigid and difficult to remove with one hand. Where the prosthetic device is a prosthetic leg, the prosthetic leg may make it difficult for the user to put on and take off pants because the user is left to take the entire prosthetic device off before putting on or taking of pants. Alternatively, the user may attempt to slide the pants over the attached prosthetic device. This may prove difficult as the shoe and leg portions of the prosthetic device form a 90° angle, making such a task difficult if not impossible. Further, some devices do not accommodate different types of users' feet and shoes and consequently it may not be easy to adjust these devices for the individual types of shoes.

Also, existing prosthetic foot devices do not allow the user to easily adjust the heel height of their prosthetic devices when changing into different shoes, because this too may involve tools to adjust the expensive and complex components of the leg pylon. This can be inconvenient for the amputee to reach in order to make the adjustment. Even further, if the user were to adjust the prosthetic in this manner, he or she may be adjusting the device incorrectly and/or contrary to how a trained doctor and/or prosthetists would adjust the device. Improper adjustment of the prosthetic device by a user may result in further debilitating illnesses associated with the user's muscle and bone structure.

The prosthetic device described herein is a device which, when attached to an amputee's prosthetic limb will provide a convenient and elegant solution to the difficulties that many amputees face during their daily routine. The device features two sections, an upper member and a lower member in which the upper member is selectively coupled to the bottom of a prosthetic limb, and the lower member is selectively coupled to the upper member. The lower member may have a prosthetic foot coupled to it such that the foot may be inserted into a shoe. The lower member may be quickly released from the upper member and may allow for any number of different lower members to be attached thereto and thereby allow a user to attach a number of different types of shoes to the user's prosthetic device. The prosthetic device may further comprise a conical mounting surface between the upper and lower members that distributes the load placed on the two members so that the device may support the weight of the user as well as additional weight that the user may carry. This may provide the amputee with many conveniences in his daily life.

The present application, therefore, describes a prosthetic device comprising an upper member and a lower member having a conical terminating section, the conical terminating section fitting into a conical void defined in the upper member.

The present application further describes a prosthetic device kit, comprising an upper member and a number of lower members each having a conical terminating section in which the conical terminating sections of each lower member fit into a conical void defined in the upper member.

The present application further describes an interlocking prosthetic device comprising an upper member selectively coupled at a proximal end to a terminating end of a prosthetic device and a lower member selectively coupled to a distal end of the upper member in which the distal end of the upper member comprises a conical void defined therein to receive a conical proximal end of the lower member.

In the following description, for purposes of explanation, numerous specific details are set forth in order to provide a thorough understanding of the present systems and methods, the methods not forming part of the invention as claimed. It will be apparent, however, to one skilled in the art that the present apparatus, systems and methods may be practiced without these specific details. Reference in the specification to "an example" or similar language indicates that a particular feature, structure, or characteristic described in connection with that example is included as described, but may not be included in other examples.

In the present specification and in the appended claims, the term "prosthetic device" is meant to be understood broadly as any mechanical device by which an amputee uses as a replacement for a missing body part. Therefore, although the present application describes a prosthetic device that replaces a user's leg or single piece prosthetic device, the present application contemplates the use of the below described upper and lower members for any other missing body part including, for example, an upper arm, a lower arm, or a finger, among others.

Additionally, as used in the present specification and in the appended claims, the term "a number of" or similar language is meant to be understood broadly as any positive number comprising 1 to infinity; zero not being a number, but the absence of a number.

The invention is defined in independent claim 1, preferred embodiments are disclosed in the dependent claims.

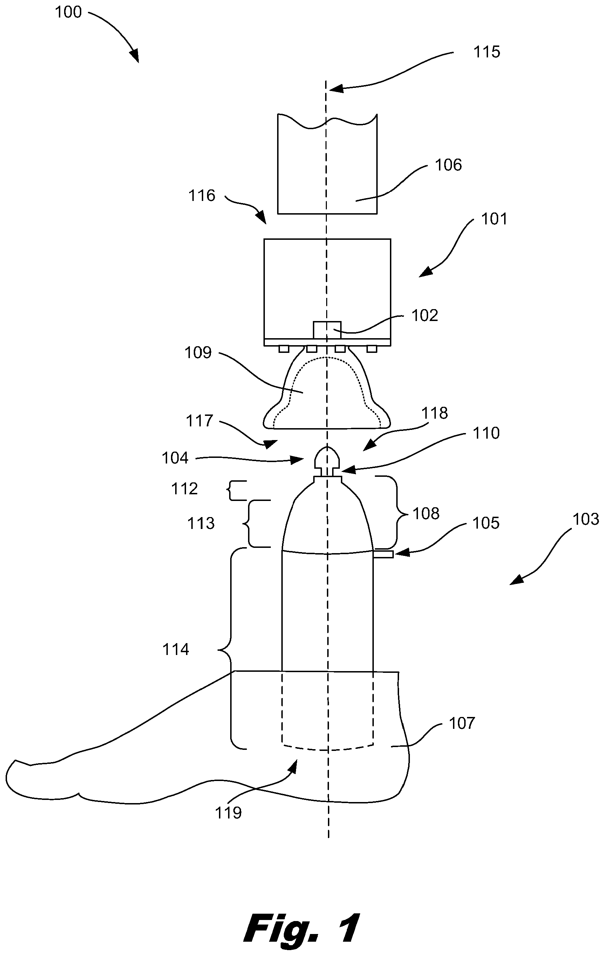

Turning now to the figures, Fig. 1 shows a front elevation view of the prosthetic device (100) according to one example of the principles described herein. The prosthetic device may comprise an upper member (101) and a lower member (103). The upper member (101) may be selectively coupled to a distal end of a prosthetic limb (106) at a proximal end (116) of the upper member (101) and to the lower member (103) at a distal end (117) of the upper member (101). The lower member (103) may be selectively coupled to the upper member (101) at a proximal end (118) of the lower member (103) and a prosthetic foot or shoe (107) on a distal end (119) of the lower member (103).

The upper member (101) comprises a conical receiving void (109) that is designed to house and accept a conical terminating section (108) at the proximal end (118) of the lower member (103). Both the conical receiving void (109) and conical terminating section (108) may be comprised of high strength materials so that they are capable of supporting a significant load, including the weight of the user, additional weight carried by the user, and the components of the prosthetic device (100). In one example, the conical receiving void (109) of the upper member (101) has an exterior bell shape. In one example, the conical receiving void (109) is constructed of high strength metal and is between 3 and 7 mm thick with the bottom of the conical receiving void (109) being the thickest part.

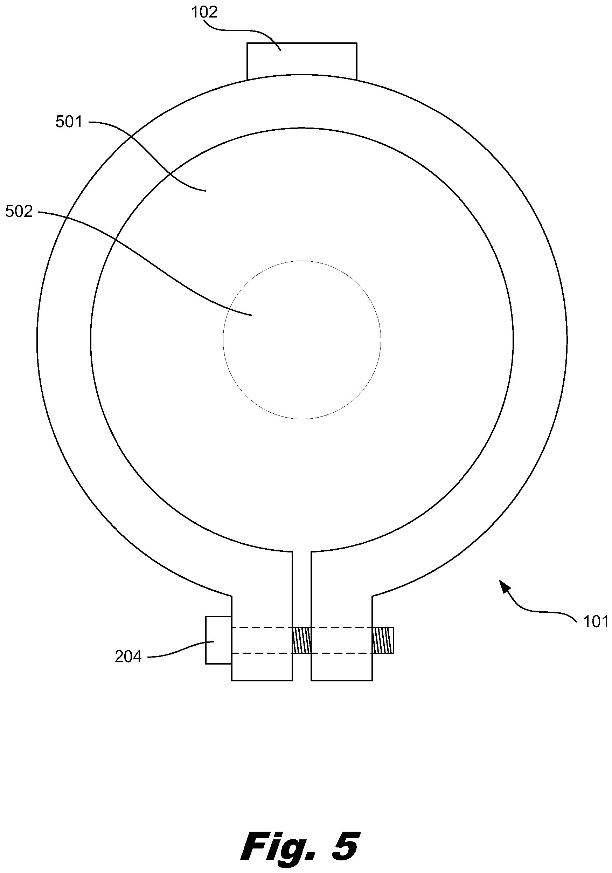

The upper member (101) may further comprise a release button (102) that may be operated using a single hand. This may allow the user to selectively release the lower member (103) from the upper member (101) when, for example, the user is getting dressed or undressed or when the user is switching to a different type of shoe attached to a separate lower member (103). The upper member (101) may further comprise a prosthetic device coupler (Fig. 5, 501) generally in the shape of a cylinder. The prosthetic device coupler (Fig. 5, 501) will be described in more detail below.

Although Fig. 1 shows positioning characteristics of the prosthetic limb (106), prosthetic device (100), and prosthetic foot/shoe (107) relative to each other, in one example the prosthetic device may be rotated about a common axis (115) of the prosthetic limb (106), prosthetic device (100), and prosthetic foot/shoe (107). Thus, the present specification contemplates a prosthetic device (100) where features thereof may be positioned around the common axis (115) but still serve similar functions as described herein.

As described above, the lower member (103) comprises a generally cylindrical shape with a conical terminating section (108) at the proximal end (112). The lower member (103) may be made of a high strength material designed to support a relatively substantial amount of weight; in some examples exceeding that of the user's body weight. In one example, the walls of a cylindrical section (114) of the lower member (103) are between 1 mm and 3 mm thick through the entire section, with the cylinder having a total diameter of 3 cm. Other examples of the cylindrical section (114) may comprise examples of larger or smaller wall thickness or larger or smaller diameters to fit the type, weight, and activity of the user implementing the prosthetic device (100). Other examples comprise varying the thicknesses or diameters of the cylindrical section (114) of the lower member (103) and all parts of the prosthetic device (103) in order to increase or decrease the load bearing capacity of the lower member (103) while increasing or decreasing the total weight of the prosthetic device (100).

The cylindrical section (114) can have any height that may be appropriate by the patient's prosthetists, with one example measuring between 3 and 4 cm high. At the top of the cylindrical section (114) is the self-aligning feature (105), which is used to orient the lower member (103) correctly when mating with the upper member (101). The self-aligning feature (105) may comprise a small protrusion extending radially from the surface of the lower member (103). In one example the self-aligning feature (105) is a rod approximately 3 to 5 mm long with a diameter of approximately 3 mm.

The conical terminating section (108) of the lower member (103) may have 2 degrees of taper, with a first part of the conical section (112) being steeper than a second part of the conical section (113). In one example, the walls of the conical terminating section (108) may be between 3 and 6 mm thick so as to provide sufficient load bearing strength for the prosthetic device (100). The conical shape of the conical terminating section (108) also aids in simplifying the mating process of the lower member (103) to the upper member (101). Coupled to the top the conical section (108) there is a locking head (104).

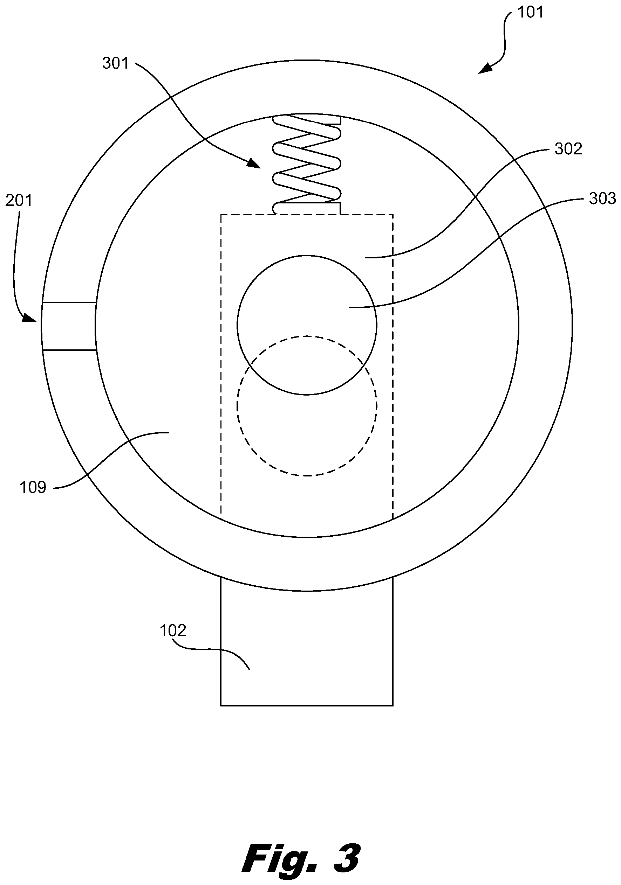

The locking head (104) may engage with a locking clip (Fig. 3, 301) located on the upper member (101). The locking head (104) and locking clip (Fig. 3, 301) together secure the lower member (103) to the upper member (101) when the conical terminating section (108) of the lower member (103) is inserted into the conical receiving void of the upper member (101). In one example, the locking head (104) is a hemispherical body mounted atop a small cylindrical pylon extending from the apex of the conical section (113) of the upper member (101). As such, the locking clip (Fig. 3, 301) may engage with a space (110) defined between the hemispherical body and the apex of the conical section (113).

In one example, the pylon has a diameter of between approximately 6 mm and 8 mm and a height of between approximately 5 mm and 7 mm. In this example, the hemispherical body atop the pylon has a larger diameter of between approximately 12 mm and 14 mm, and vertical radius of between approximately 8 mm and 10 mm. The locking head (104) allows the locking clip (Fig. 3, 301) of the upper member (101) to secure itself around the small cylindrical pylon of the locking head (104), being secured by the conical section below, and the elongated hemisphere above. The circular profile of the locking head (104) also simplifies the mating process by allowing the locking head (104) to enter into the locking void of the upper member (101) from any angle.

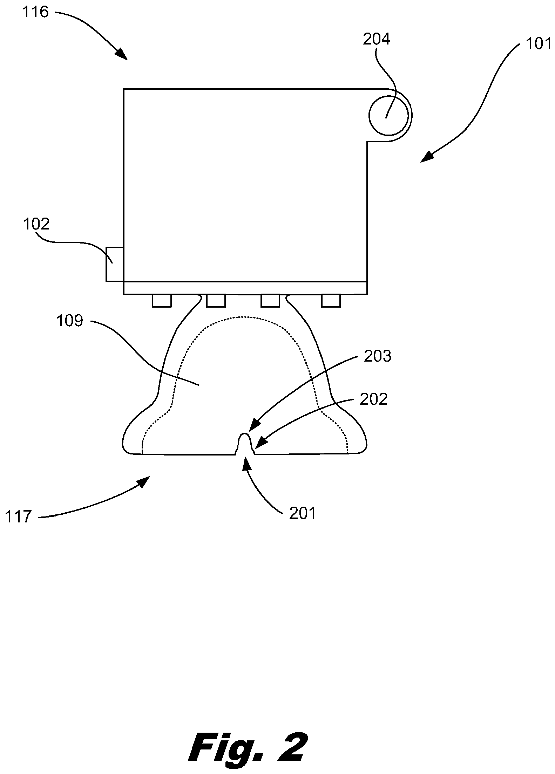

Fig.2 shows a rotated elevation view of the upper member (101). Shown in Fig. 2 is a guiding feature (201) which works in conjunction with the self-aligning feature (105) on the lower member (103) to guide the locking head (104) into the locking clip (Fig. 3, 302) and properly align the upper member (101) with the lower member (103). The guiding feature (201) is composed of a guiding track (202) and a guiding notch (203). The guiding track (202) is a slightly angled taper which will guide the self-aligning feature (104) of the lower member (103) around the edge of the conical receiver void (109) until the self-aligning feature (104) securely houses itself in the guiding notch (203) found in the guiding feature (201). In one example the guiding track (202) may run 10 to 20 mm along the circumference of the conical receiver void (109). In other examples the guiding track (202) may run along the entire circumference of the conical receiver void (109) such that, as the lower member (103) is engaged with the upper member (101), the slope of the circumference of the conical receiver void (109) created by the guiding track (202) will guide the self-aligning feature (105) into the guiding notch (203) wherever the self-aligning feature (105) comes in contact with the circumference of the conical receiver void (109). The guiding notch (203) is a cylindrical cutout from circumference of the conical receiver void (109) which is sufficiently sized to fit and secure the self-aligning feature (105) therein.

The upper member (101) may further comprise a tightening screw (204) to secure the distal end of a prosthetic limb (106) with the upper member (101). In one example, the tightening screw (204) may comprise a screw traversing through a separated portion of the upper member (101) such that tightening of the screw closes the separated portion of the upper member (101) thereby reducing the overall diameter of the upper member (101). In one example, the tightening screw (204) may be a quick release device that allows easy attachment of the upper member (101) to the prosthetic limb (106). In another example, the tightening screw (204) may comprise a screw that is not easily adjusted by a user of the prosthetic device (Fig. 100). In this example, the screw may comprise an engagement head such that a tool not readily accessible to a common consumer is implemented in order to manipulate the screw. In this example, this may be done so as to prevent the user of the prosthetic device (Fig. 1, 100) from adjusting the upper member (101)/prosthetic limb (106) interface thereby preventing improper adjustment of the prosthetic device (100) by the user. In another example, the upper member (101) may be adhesively bonded to the prosthetic limb (106). In this example, the tightening screw (204) is not used to secure the upper member (101) to the prosthetic limb (106) thereby eliminating weight and additional components.

Fig. 3 shows a bottom view cutout of the locking features of the upper member (101). Fig. 3 shows the functions of the locking mechanism that secure the locking head (104) of the upper member (101). The locking head (104) is shaped and sized so that it can pass through a circular opening of a circular receiver void (303). Upon entering the cavity, the hemispherical top of the locking head (104) will exert force on an upper locking clip (302) such that the force depresses a spring (301) biased radially inward relative to a common axis (115) of the upper (101) the lower members (103). Once the hemisphere of the locking head (104) has pushed past the upper locking clip (302), the spring (301) may extend the locking clip (302) back into its original position to secure and edge of the upper locking clip (302) against the pylon of the locking head (104), preventing the separation of the lower member (103) from the upper member (101) and securely mating the two together. To remove the upper member (101) from the lower member (103), the release button (102) may be depressed. The depression of the release button (102) pushes the upper locking clip (302) back away from the pylon and compresses the spring (301). This releases the tension on the lower member's (103) locking head (104) and allows for the easy separation of the two members (Fig. 1, 101 and 103). The release button (102) uses minimal force to depress, such that it may be operated with one hand or even one finger, freeing the other hand to perform other tasks.

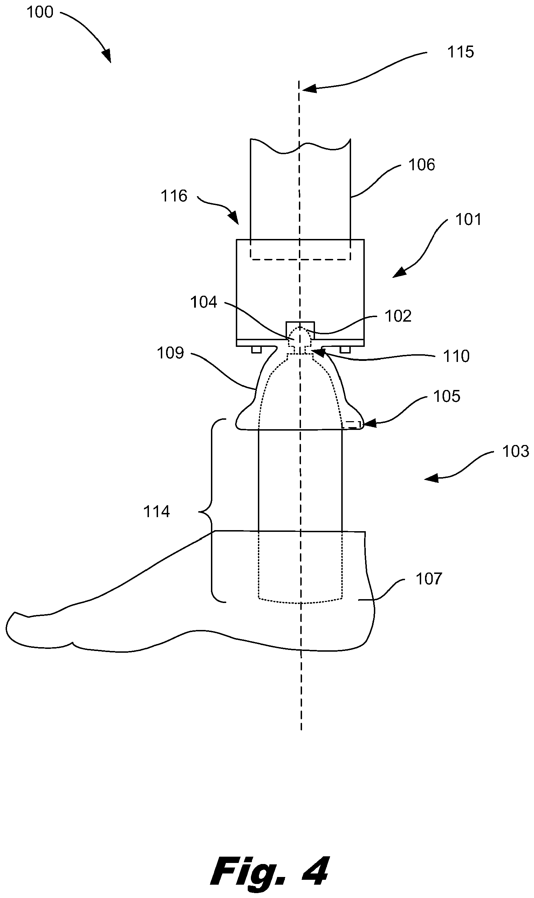

Fig. 4 is a front elevation cutout view of the prosthetic device (100) which further demonstrates the mating position of the upper member (101) and lower member (103). Fig. 4 shows one example of the contact points between the conical terminating member (108) of the lower member (103) and the conical receiving void (109) of the upper member (101). The cone shape of the two members effectively distributes the weight across the entire area of the interface. This figure also shows how the locking head (104) is secured by the locking clip (302).

Fig. 5 is a top view of the upper member (101) shown in Fig. 1. Fig. 5 shows a void into which a user may mount a prosthetic limb (Fig. 1, 106) into in order to selectively attach the prosthetic limb (Fig. 1, 106) to the upper member (101). A prosthetic limb mounting area (501) may comprise a cylindrical impression between 2 and 3 cm deep, with a diameter of approximately 3 cm in one example. However, the mounting area (501) may be any size in order to properly mount any prosthetic limb to the upper member (101). The upper member (101) may comprise a small cylindrical housing (502) which houses the locking head (104) of the lower member (103) when the lower member (103) and upper member (101) are coupled together. The housing (502) may extend between .5 cm and 1 cm above the mounting area (501) and may have a diameter of between 1 cm and 1.5 cm.

The prosthetic limb (106), being a hollow cylindrical body, may encompass the housing (502) and then, in one example, be able to be tightened onto the upper member (101) using a tightening screw (Fig. 2, 204) or a quick release mechanism. The prosthetic limb mounting area (501) may be slightly larger in diameter than the diameter of the cylindrical body of the prosthetic limb (Fig. 1, 106) such that the tightening screw (Fig. 2, 204), when fastened, will compress against the prosthetic limb (Fig. 1, 106) closing a tightening allowance between the prosthetic limb and the prosthetic limb mounting area (501). The tightening allowance may be a small gap approximately 1 mm wide. This allows the mounting area (501) to securely fasten to the prosthetic limb (Fig. 1, 106). As described above, in another example, the upper member (101) may be adhesively bonded to the prosthetic limb (106), however, the prosthetic limb mounting area (501) may still be formed so as to receive the prosthetic limb (Fig. 1, 106).

The prosthetic device (Fig. 1, 100) described above may further be arranged in the form of a kit. In one example, the kit may comprise a single upper member (101) with a number of lower members (103). The upper member (101) may be coupled permanently or semi- permanently to a portion of the user's prosthetic device (Fig. 1, 100). Often a prosthetic device (Fig. 1, 100) comprises a cuff portion that wraps around a remaining portion of an amputee's limb. The cuff may be coupled to a rigid single tube that further couples to a prosthetic foot or shoe. The upper member described above may be attached to a terminal end of the tube. The attachment of the upper member (101) to the tube may be done by a trained technician and as such may be set permanently or semi-permanently to the tube of the existing prosthetic.

During this adjustment, the technician may further match the number of lower members (Fig. 1, 103) to the upper member (Fig. 1, 101). As described above, each lower member (Fig. 1, 103) comprises a self-aligning feature (Fig. 1, 104) that mates with a guiding notch (Fig. 2, 203) on the upper member. As such, the technician may adjust any prosthetic foot or shoe (Fig. 1, 107) that is to be attached to the lower member (Fig. 1, 103) such that when the user couples the lower member (Fig. 1, 103) with the foot or shoe attached) to the upper member (Fig. 1, 101) the alignment is made to best accommodate the feel of the user. In this example, every lower member (Fig. 1, 103) the user couples to the upper member (Fig. 1, 101) will be properly aligned due to the self-aligning feature (Fig. 1, 104) and the adjustments made by the technician with regards to the upper member/tube and lower member/shoe interfaces.

The specification and figures describe a prosthetic device that provides a user with the ability to detach the device from a prosthetic limb and self-align the device to the limb when re-attaching. This allows a user of the device to operate the release button (Fig. 1, 102) to detach the upper member (Fig. 1, 101) from the lower member (Fig. 1, 103) with one hand. Additionally, the conical shape of the interface between the two members (Fig. 1, 101, 103) provides for better weight distribution thereby allowing for less wear and tear on the device and greater comfort for the user. Still further, the spherical locking head (Fig. 1, 104) provide for easier insertion of a lower member (103) into the upper member (Fig. 1, 101) such that a user does not have to be as precise when engaging the two members. Still further, the device allows a user to selectively detach and reattach a number of lower members. These lower members (Fig. 1, 103) may be further coupled to a number of different foot wear such that a user may decouple the lower member (Fig. 1, 103) from the upper (Fig. 1, 101) when exchanging one type of shoe (i.e. dress shoe) for another type of shoe (i.e. running shoe). Additionally, by allowing the user to selectively remove the lower member (Fig. 1, 103) from the upper member (Fig. 1, 101) the user may more comfortably remove any pants or other clothing without having to remove their prosthetic device as well.

The preceding description has been presented to illustrate and describe examples of the principles described. This description is not intended to be exhaustive or to limit these principles to any precise form disclosed. Many modifications and variations are possible in light of the above disclosure.

## Claims

1. A prosthetic device (100) comprising:
an upper member (101) comprising:
a guiding feature (201), comprising a sloped guiding track (202) and a notch (203); and
a lower member (103) comprising:
a conical terminating section (108) formed at a proximal end (118) of the lower member, wherein the conical terminating section is adapted to fit into a conical void (109) defined in the upper member and comprises a first conical part (112) which has a steeper angle than a second conical part (113) relative to a common axis (115) of the upper and lower members;
a locking head (104) which is coupled to a proximal end of the first conical part and is adapted to fit into a conical void defined in the upper member; and
a self-aligning feature (105) comprising a protrusion extending radially from a section of the lower member and extending perpendicular to the common axis of the upper and lower members, in which the protrusion prevents the lower member from mating at an incorrect rotational position to the upper member;
wherein contact of the self-aligning feature with the sloped guiding track as the conical terminating section is fitted into the conical void causes the lower member to rotate until the self-aligning feature is seated within the notch.

2. The prosthetic device of claim 1, in which the upper member is adapted to selectively attach to a terminating member of a prosthetic limb (106).

3. The prosthetic device of claim 1, in which the sloped guiding track runs circumferentially around the edge of the conical void (109) of the upper member.

4. The prosthetic device of claim 3, wherein the guiding track runs 20 mm around the edge of the conical void.

5. The prosthetic device of claim 4, wherein the notch is cut out in a midway point along the sloped guiding feature.

6. The prosthetic device of claim 1, in which the upper member further comprises a locking mechanism (302, 301) to a securely lock the upper member to the lower member.

7. The prosthetic device of claim 6, in which the locking mechanism comprises a spring loaded clip (302) that locks the lower member to the upper member when the lower member is inserted into the upper member.

## Patentansprüche

1. Prothesenvorrichtung (100), die Folgendes umfasst:
ein oberes Element (101), das Folgendes umfasst:
ein Führungsmerkmal (201), umfassend eine schräge Führungsbahn (202) und eine Einkerbung (203); und
ein unteres Element (103), das Folgendes umfasst:
einen konischen Abschlussabschnitt (108), der an einem proximalen Ende (118) des unteren Elements gebildet ist, wobei der konische Abschlussabschnitt dazu angepasst ist, in einen in dem oberen Element definierten konischen Hohlraum (109) zu passen,
und einen ersten konischen Teil (112) umfasst, der einen steileren Winkel als ein zweiter konischer Teil (113) relativ zu einer gemeinsamen Achse (115) des oberen und des unteren Elements aufweist;
einen Verriegelungskopf (104), der an ein proximales Ende des ersten konischen Teils gekoppelt ist und dazu angepasst ist, in einen in dem oberen Element definierten konischen Hohlraum zu passen; und
ein Selbstausrichtungsmerkmal (105), umfassend einen Vorsprung, der sich von einem Abschnitt des unteren Elements radial erstreckt und sich senkrecht zu der gemeinsamen Achse des oberen und des unteren Elements erstreckt, wobei der Vorsprung verhindert, dass das untere Element in einer falschen Drehlage mit dem oberen Element zusammenpasst;
wobei der Kontakt des Selbstausrichtungsmerkmals mit der schrägen Führungsbahn, wenn der konische Abschlussabschnitt in den konischen Hohlraum eingesetzt wird, bewirkt, dass sich das untere Element dreht, bis das Selbstausrichtungsmerkmal in der Einkerbung sitzt.

2. Prothesenvorrichtung nach Anspruch 1, wobei das obere Element zum selektiven Anbringen an einem Abschlusselement eines prothetischen Körperglieds (106) angepasst ist.

3. Prothesenvorrichtung nach Anspruch 1, wobei die schräge Führungsbahn in Umfangsrichtung um den Rand des konischen Hohlraums (109) des oberen Elements verläuft.

4. Prothesenvorrichtung nach Anspruch 3, wobei die Führungsbahn 20 mm um den Rand des konischen Hohlraums verläuft.

5. Prothesenvorrichtung nach Anspruch 4, wobei die Einkerbung an einer mittleren Stelle entlang des schrägen Führungsmerkmals ausgeschnitten ist.

6. Prothesenvorrichtung nach Anspruch 1, wobei das obere Element ferner einen Verriegelungsmechanismus (302, 301) zum sicheren Verriegeln des oberen Elements an dem unteren Element umfasst.

7. Prothesenvorrichtung nach Anspruch 6, wobei der Verriegelungsmechanismus einen federgespannten Clip (302) umfasst, der das untere Element an dem oberen Element verriegelt, wenn das untere Element in das obere Element eingesteckt wird.

## Revendications

1. Dispositif prothétique (100) comprenant :
un élément supérieur (101) comprenant :
un élément de guidage (201), comprenant une piste de guidage inclinée (202) et une encoche (203) ; et
un élément inférieur (103) comprenant :
une section de terminaison conique (108) formée au niveau d'une extrémité proximale (118) de l'élément inférieur, dans lequel la section de terminaison conique est conçue pour s'ajuster dans un vide conique (109) défini dans l'élément supérieur et comprend une première partie conique (112) qui a un angle plus aigu qu'une deuxième partie conique (113) par rapport à un axe commun (115) des éléments supérieur et inférieur ;
une tête de verrouillage (104) qui est couplée à une extrémité proximale de la première partie conique et est conçue pour s'ajuster jusque dans un vide conique défini dans l'élément supérieur ; et
un élément d'auto-alignement (105) comprenant une saillie s'étendant de manière radiale depuis une section de l'élément inférieur et s'étendant perpendiculaire à l'axe commun des éléments supérieur et inférieur, dans lequel la saillie empêche l'élément inférieur de s'apparier au niveau d'une position de rotation incorrecte à l'élément supérieur ;
dans lequel l'entrée en contact de l'élément d'auto-alignement avec la piste de guidage inclinée quand la section de terminaison conique est ajustée jusque dans le vide conique amène l'élément inférieur à tourner jusqu'à ce que l'élément d'auto-alignement repose au sein de l'encoche.

2. Dispositif prothétique selon la revendication 1, dans lequel l'élément supérieur est conçu pour s'attacher sélectivement à un élément de terminaison d'un membre prothétique (106).

3. Dispositif prothétique selon la revendication 1, dans lequel la piste de guidage inclinée s'étend de manière circonférentielle autour du bord du vide conique (109) de l'élément supérieur.

4. Dispositif prothétique selon la revendication 3, dans lequel la piste de guidage s'étend sur 20 mm autour du bord du vide conique.

5. Dispositif prothétique selon la revendication 4, dans lequel l'encoche est découpée au niveau d'un point de mi-parcours le long de l'élément de guidage incliné.

6. Dispositif prothétique selon la revendication 1, dans lequel l'élément supérieur comprend en outre un mécanisme de verrouillage (302, 301) pour verrouiller solidement l'élément supérieur à l'élément inférieur.

7. Dispositif prothétique selon la revendication 6, dans lequel le mécanisme de verrouillage comprend une pince à ressort (302) qui verrouille l'élément inférieur à l'élément supérieur quand l'élément inférieur est inséré jusque dans l'élément supérieur.
